# EUROPÄISCHE PATENTSCHRIFT

(11) **EP 0 332 075 B1**
(45) Veröffentlichungstag und Bekanntmachung des Hinweises auf die Patenterteilung: **02.06.1993**
(21) Anmeldenummer: 89103747.5
(22) Anmeldetag: 03.03.1989
(51) Int. Cl.: C07D 233/64

(54) **Verfahren zur Herstellung von acylierten Imidazolen**
Process for the preparation of acylated imidazoles
Procédé pour la fabrication d'imidazoles acylés

(30) Priorität: 11.03.1988 DE 3808071
(43) Veröffentlichungstag der Anmeldung: 13.09.1989
(73) Patentinhaber: BASF Aktiengesellschaft, 67063 Ludwigshafen (DE)
(72) Erfinder: Hesse, Michael, Dr., D-6700 Ludwigshafen (DE); Hoelderich, Wolfgang, Dr., D-6710 Frankenthal (DE); Dockner, Toni, Dr., D-6701 Meckenheim (DE); Koehler, Hermann, Dr., D-6711 Beindersheim (DE)

(56) Entgegenhaltungen:
- EP-A- 0 116 205
- EP-A- 0 156 644
- EP-A- 0 300 324
- DE-A- 3 228 266
- HELVETICA CHIMICA ACTA, Band 69, Fasc. 8, Nr. 294, 1976, Seiten 2738-2752

## Beschreibung

Die Anmeldung betrifft ein Verfahren zur Herstellung von acylierten Imidazolen durch Direktacylierung von entsprechenden unsubstituierten oder substituierten Imidazolen mit gängigen Acylierungsmitteln an sauren Metalloxiden und Phosphaten.

Bis jetzt waren direkte C-Alkylierungen von Imidazolen in Friedel-Crafts-Reaktionen nicht bekannt und wurden auch nicht für ausführbar gehalten (A.R. Katritzky, C.W. Rees, Comprehensive Heterocyclic Chemistry, Vol. 5, S. 402 (1984), Pergamon Press; K. Hofmann. The Chemistry of Heterocyclic Compounds, Vol. 6; Imidazole and its derivatives, Part I, S. 49 und S. 59 (1953)), Interscience Publishers).

Daher war man gezwungen, auf andere Möglichkeiten der Darstellung auszuweichen, z.B. die photochemische Umlagerung von N-Acetylimidazolen (J. L. La Mattina et al., J. Org. Chem. 48, 897-8 (1983)), die Umsetzung von 4-Formylimidazolen mit einem Grignard-Reagens und nachfolgende Oxidation (z.B. R. Paul et al., J, Med. Chem. 28, 1704-16 (1985)) oder die Hydrogenolyse eines 4-Acylamino-5-methylisoxazols (EP 156 644, Pfizer).

Diese bekannten Verfahren sind jedoch-aufwendig und im technischen Maßstab nicht durchführbar.

Es wurde nun überraschenderweise ein Verfahren zur Herstellung von acylierten Imidazolen der Formel I 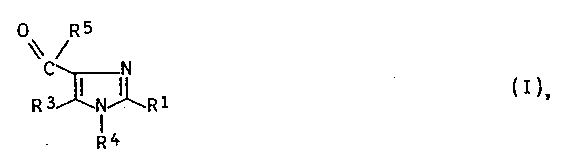
in der R¹ und R³ = H, Alkyl mit 1 bis 12 Kohlenstoffatomen, Alkenyl mit bis zu 12 Kohlenstoffatomen, Aryl- oder Aralkyl- oder Alkylarylreste,
R⁴ = H-, Alkyl mit 1 bis 12 Kohlenstoffatomen, Alkenyl mit bis zu 12 Kohtenstoffatomen, Aryl-, Aralkyl- oder Alkytarylreste, sowie R⁵ = Alkyl mit 1 bis 12 Kohlenstoffatomen, Alkenyl mit bis zu 12 Kohlenstoffatomen, Aryl-, Aralkyl-, Alkylaryl- oder _Carboxyreste bedeuten, gefunden, welches dadurch gekennzeichnet ist, daß man Imidazole der Formel II 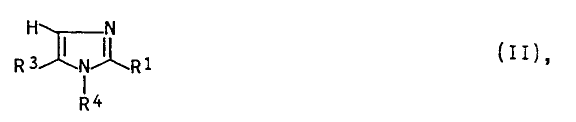
in der R¹ und R³ = Alkyl mit 1 bis 12 Kohlenstoffatomen, Alkenyl mit bis zu 12 Kohlenstoffatomen, Aryl-, Aralkyl- oder Alkylarylreste und R⁴ = H, Alkyl mit 1 bis 12 Kohlenstoffatomen, Alkenyl mit bis zu 12 Kohlenstoffatomen, Aryl-, Aralkyl- oder Alkylarylreste bedeuten, mit gängigen Acylierungsmitteln der Formel (III) 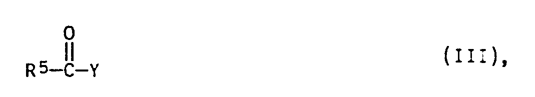
in der R⁵ die obengenannte Bedeutung hat und Y = Halogenid, Alkoxy-, Acyloxy- oder Hydroxyreste bedeutet, in Gegenwart von sauren Metalloxiden der zweiten bis vierten Haupt- und dritten bis sechsten Nebengruppe oder deren Gemische, die gegebenenfalls durch Phosphor- oder Borsäure getränkt sind, ohne Zeolithstruktur und/oder Aluminiumphosphate, Siliciumaluminiumphosphate, Siliciumeisenaluminiumphosphate, Cerphosphate, Zirkonphosphate, Borphosphate, Eisenphosphate, Strontiumphosphate oder deren Gemische in der Gasphase umsetzt.

Die Umsetzung von 2-Methylimidazol mit Essigsäureanhydrid zu 4-Acetyl-2-methylimidazol an einem Katalysator kann man beispielsweise durch folgende Formelgleichung wiedergeben:

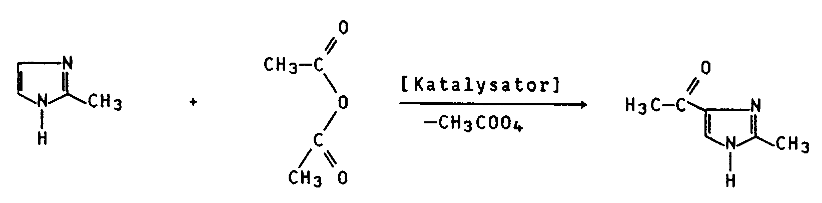

Die analoge Umsetzung von 4-Methylimidazol liefert in der Hauptsache 5-Acetyl-4-methylimidazol, daneben aber auch 2-Acetyl-4-methylimidazol.

Als Ausgangsstoffe für das erfindungsgemäße Verfahren der oben angegebenen Formel (II) sind z.B. geeignet Imidazol, 2-Methylimidazol, 2-Ethylimidazol, 2-Propylimidazol, 2-Isopropylmidazol, 2-Decylimidazol, 2-Dodecylimidazol, 1-Methylimidazol, 4-Methylimidazol, 2-Ethyl-4-methylimidazol, 4-n-Butylimidazol, 2-Isopropylimidazol, 2-Isopropyl-4-ethylimidazol, 2,4-Di-n-butylimidazol, 2-Phenyl-, 4-Phenylimidazol und 4-Benzylimidazol.

Als Acylierungsmittel sind z.B. Essigsäurechlorid, Essigsäure, Essigsäureanhydrid, Essigsäuremethylester, Essigsäureethylester, Essigsäureamid, Propionsäurechlorid, Propionsäure, Propionsäureanhydrid, Buttersäurechlorid, Buttersäure, Buttersäureanhydrid, Isobuttersäure Isobuttersäurechlorid, Isobuttersäureanhydrid, Crotonsäure, Crotonsäureanhydrid, Isoprensäure, Valeriansäure, Valeriansäurechlorid, Capronsäure, Capronsäureester, Pivalinsäure, Pivalinsäurechlorid, Acrylsäure, Acrylsäuremethylester, Methacrylsäure, Methacrylsäureester, Penten-3-säuremethylester, Penten-2-säuremethylester, Penten-4-säuremethylester, Sorbinsäure, Sorbinsäuremethylester, Benzoesäure, Benzoesäurechlorid, Benzoesäuremethylester, o-, m-, p-Fluorbenzoesäure, o-, m-, p-Fluorbenzoesäurechlorid, o-, m-, p-Chlorbenzoesäure, o-, m-, p-Methylbenzoesäure, o-, m-, p-Methylbenzoesäurechlorid, o-, m-, p-Methoxybenzoesäure, o-, m-, p-Methoxybenzoesäurechlorid, o-, m-, p-Isopropylbenzoesäure, o-, m-, p-Isopropylbenzoesäurechlorid, 2,3-Dimethyl-, 2,3-Difluor-, 2,3-Dichlor-, 2,3-Dimethoxybenzoesäure bzw. -benzoesäurechlorid, Phenylessigsäure, Phenylessigsäurechlorid, Phenylessigsäureanhydrid, Zimtsäure, Zimtsäurechlorid, Malonsäure, Malonsäureanhydrid, Phthalsäure, Phthalsäureanhydrid, Terephthalsäure und Terephthalsäurechlorid geeignet.

Als Katalysatoren können z.B. saure Metalloxide ohne Zeolithstruktur wie die sauer wirkenden Oxide von Elementen der II. bis IV. Hauptgruppe sowie der III. bis VI. Nebengruppe des periodischen Systems, insbesondere Siliciumdioxid in Form von Kieselgel, Kieselgur, Quarz, weiterhin Titandioxid, Zirkondioxid, Phosphoroxide, Vandiumpentoxid, Nioboxid, Bortrioxid, Aluminiumoxid, Chromoxide, Molybdänoxide, Wolframoxide oder Gemische dieser Oxide eingesetzt werden. Eine Modifizierung mit Metallen oder Säuren ist möglich.

Auch mit Phosphorsäure oder Borsäure getränkte Katalysatoren ohne Zeolithstruktur können eingesetzt werden. Phosphorsäure oder Borsäure wird auf SiO₂-, Al₂O₃- oder Bims-Träger, z.B. durch Auftränken oder Versprühen aufgebracht. Ein phosphorsäurehaltiger Katalysator kann beispielsweise durch Auftränken von H₃PO₄- oder NaH₂PO₄- oder Ha₂HPO₄-Lösung auf SiO₂ und anschließende Trocknung bzw. Calcination erhalten werden. Phosphorsäure kann aber auch mit Kieselgel zusammen in einem Spühturm versprüht werden; danach schließt sich eine Trocknung und meist eine Calcination an. Phosphorsäure kann auch in einer Imprägniermühle auf das Trägermaterial aufgesprüht werden.

Weitere Katalysatoren für die Acylierung von Imidazolen sind Phosphate, insbesondere Aluminiumphosphate, Siliciumaluminiumphosphate, Silciumeisenaluminiumphosphate, Cerphosphate, Zirkonphosphate, Borphosphate, Eisenphosphate, Strontiumphosphate oder deren Gemische.

Als Phosphat-Katalysatoren kann man gefällte Aluminiumphosphate einsetzen. Man erhält ein derartiges Aluminiumphosphat, wenn man 92 g Diammoniumhydrogenphosphat in 700 ml Wasser löst und zu dieser Lösung 260 g Al(NO₃)₃ x H₂O in 700 ml Wasser über 2 h zutropft. Dabei wird der pH-Wert durch gleichzeitige Zugabe von 25 %iger NH₃-Lösung bei pH 8 gehalten. Der entstandene Niederschlag wird 12 h nachgerührt, dann abgesaugt und ausgewaschen. Er wird bei 60°C/16 h getrocknet.

Borphosphate, die als Katalysatoren für das erfindungsgemäße Verfahren geeignet sind, kann man beispielsweise durch Mischen und Kneten von konzentrierter Borsäure und Phosphorsäure und durch anschließende Trocknung und Calcination in Inertgas-, Luft- oder Dampfatmosphäre bei 250 bis 650°C, vorzugsweise 300 bis 500°C, herstellen.

Als Aluminiumphosphat-Katalysatoren werden für das erfindungsgemäße Verfahren, insbesondere unter hydrothermalen Bedingungen, synthetisierte Aluminiumphosphate eingesetzt. Geeignete Aluminiumphosphate sind z.B. APO-5, APO-9, APO-11, APO-12, APO-14, APO-21, APO-25, APO-31 und APO-33.

AlPO₄-5 (APO-5) kann synthetisiert werden, indem man Orthophosphorsäure mit Pseudoboehmit (Catapal SB R) in Wasser homogen mischt; zu dieser Mischung Tetrapropylammoniumhydroxid gibt und diese danach bei etwa 150°C 20 bis 60 h unter autogenem Druck in einem Autoklaven umsetzt. Das abfiltrierte AlPO₄ wird bei 100 bis 160°C getrocknet und bei 450 bis 550°C calciniert.

AlPO₄-9 (APO-9) wird aus Orthophosphorsäure und Pseudoboehmit in wäßriger DABCO-Lösung (1,4-Diazabicylco-(2,2,2)-octan) bei etwa 200°C unter autogenem Druck während 200 bis 400°C synthetisiert. Verwendet man anstelle DABCO-Lösung Ethylendiamin, so gelangt man zu APO-12.

Die Synthese des AlPO₄-21 (APO-21) erfolgt aus Orthophosphorsäure und Pseudoboehmit in wäßriger Pyrrolidon-Lösung bei 150 bis 200°C unter autogenem Druck während 50 bis 200 h.

Für das erfindungsgemäße Verfahren kann man auch bekannte Siliciumaluminiumphosphate wie SPO-5, SAPO-11, SAPO-31 und SAPO-34 einsetzen. Diese Verbindungen werden durch Kristallisation aus wäßriger Mischung bei 100 bis 250°C und autogenem Druck während 2 h bis 2 Wochen hergestellt, wobei die Reaktionsmischung, die sich aus einer Silicium-, Aluminium- und Phosphorkomponente zusammensetzt, in wäßrigen aminoorganischen Lösungen umgesetzt wird.

SAPO-5 wird durch Mischen von SiO₂ suspendiert in wäßriger Tetrapropylammoniumhydroxid-Lösung mit einer wäßrigen Suspension aus Pseudoboehmit und Orthophosphorsäure und anschließende Umsetzung bei 150 bis 200°C, während 20 bis 200 h unter autogenem Druck in einem Rührautoklaven erhalten. Die Trocknung des abfiltrierten Pulvers erfolgt bei 110 bis 160°C und die Calcination bei 450 bis 550°C.

Als Siliciumaluminiumphosphat sind auch ZYT-5, ZYT-6, ZYT-7, ZYT-9, ZYT1-11 und ZYT-12 geeignet.

Um eine möglichst hohe Selektivität, hohen Umsatz sowie lange Standzeiten zu erreichen, ist es vorteilhaft, die Katalysatoren zu modifizieren.

Eine Möglichkeit der Modifizierung besteht darin, daß man das Material - verformt oder unverformt - einer Behandlung mit Säuren wie Salzsäure, Flußsäure und Phosphorsäure und/oder Wasserdampf unterwirft. Dabei geht man vorteilhaft so vor, daß man die Katalysatoren in Pulverform mit 1 n Phosphosäure 1 h bei 80°C behandelt. Nach der Behandlung wird mit Wasser gewaschen, bei 110°C/16 h getrocknet und bei 500°C/20 h calciniert. Nach einer anderen Arbeitsweise behandelt man die Katalysatoren vor oder nach ihrer Verformung mit Bindemitteln, z.B. 1 bis 3 h bei Temperaturen von 60 bis 80°C mit einer 3 bis 25 gew.%igen, insbesondere 12 bis 20 gew.%igen wäßrigen Salzsäure. Anschließend wird der so behandelte Katalysator mit Wasser gewaschen, getrocknet und bei 400 bis 500°C calciniert.

Eine besondere Ausführungsform für die Säurebehandlung besteht darin, daß man das Material vor seiner Verformung bei erhöhter Temperatur mit 0,001 n bis 2 n Flußsäure, vorzugsweise 0,05 bis 0,5 n Flußsäure durch Erhitzen unter Rückfluß über einen Zeitraum von 0,5 bis 5 h, vorzugsweise 1 bis 3 h behandelt. Nach Isolierung durch Abfiltrieren und Auswaschen des Materials wird dieses zweckmäßig bei Temperaturen von 100 bis 160°C, getrocknet und bei Temperaturen von 450 bis 600°C calciniert. Gemäß einer bevorzugten Ausführungform für die Säurebehandlung wird das Material nach seiner Verformung mit Bindemittel bei erhöhter Temperatur, zweckmäßig bei 50 bis 90°C 1 bis 3 h mit 25 gew.%iger Salzsäure, vorzugsweise mit 12 bis 20 gew.%iger Salzsäure behandelt. Anschließend wird das Material ausgewaschen, bei Temperaturen von 100 bis 160°C getrocknet und bei Temperaturen von 450 bis 600°C calciniert. Auch eine aufeinanderfolgende Behandlung mit HF und HCl ist gegebenenfalls vorteilhaft.

Eine andere Arbeitsweise besteht darin, daß man die Katalysatoren durch Aufbringen von Phosphorverbindungen, wie Trimethylphosphat, Trimethoxyphosphin, primärem, sekundärem oder tertiärem Natriumphosphat modifiziert. Als vorteilhaft hat sich die Behandlung mit primärem Natriumphosphat erwiesen. Hierbei werden die Zeolithe in Strang-, Tabletten- oder Wirbelgut-Form mit wäßriger NaH₂PO₄-Lösung getränkt, bei 110°C getrocknet und bei 500°C calciniert.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelgut eingesetzt werden.

Die erfindungsgemäße Umsetzung wird vorzugsweise in der Gasphase bei 300 bis 600°C, insbesondere bei 400 bis 550°C und einer Belastung von WHSV von 0,1 bis 20 h⁻¹, insbesondere 0,5 bis 5 h⁻¹ (g Einsatzgemisch/g Katalsator und Stunde) in einem Festbett oder Wirbelbett ausgeführt.

Es ist auch möglich, die Reaktion in der Flüssigphase in Suspension, Riesel- oder Sumpffahrweise bei Temperaturen zwischen 50 und 300°C, insbesondere zwischen 100 und 250°C und einer Belastung g Ausgangsstoff : g Katalysator = 100 : 1 bis 5 : 1, vorzugsweise 60 : 1 bis 10 : 1 durchzuführen.

Das Verfahren kann bei Normaldruck oder je nach Flüchtigkeit der Ausgangsverbindung bei vermindertem Druck oder erhöhtem Druck, vorzugsweise kontinuierlich durchgeführt werden.

Schwerflüchtige oder feste Ausgangsstoffe werden in gelöster Form, z.B. in THF-, Toluol- oder Petrolether-Lösung, eingesetzt. Im allgemeinen ist eine Verdünnung des Ausgangsstoffes mit Lösungsmitteln oder mit Inertgasen, wie N₂, Ar, He oder mit H₂O-Dampf möglich.

Nach der Umsetzung werden die acylierten Imidazole durch übliche Verfahren, z.B. durch Destillation aus dem Reaktionsgemisch isoliert; nichtumgesetztes Ausgangsgemisch wird gegebenenfalls zurückgeführt.

### Beispiele 1 bis 10

Die Reaktion in der Gasphase wird unter isothermen Bedingungen in einem Rohrreaktor (Wendel, 0,6 cm Innendurchmesser, 90 cm Länge) mindestens 6 h lang durchgeführt. Die Reaktionsprodukte werden durch übliche Verfahren abgetrennt und durch GC/MS, NMR und Bestimmung des Schmelzpunktes charakterisiert. Die quantitative Bestimmung der Reaktionsprodukte und der Ausgangsprodukte erfolgt gaschromatographisch.

Die in den nachfolgenden Anwendungsbeispielen eingesetzten Katalysatoren sind:

### Katalysator A

Handelsübliches Al₂O₃ (D 10-10®, BASF) in Form von Strangpreßlingen

### Katalysator B

Handelsübliches SiO₂ (D 11-10®, BASF) in Form von Strangpreßlingen

### Katalysator C

Im Handel erhältliches Zirkonphosphat Zr₃(PO₄)₄ wird mit einem Verformungshilfsmittel zu 2-mm-Strängen verformt, bei 110°C getrocknet und bei 500°C 16 h calciniert.

### Katalysator D

Katalysator D ist ein gefälltes Borphosphat, hergestellt indem man 49 g H₃BO₃ mit 117 g H₃PO₄ (75 %ig) in einem Kneter zusammengibt, überschüssiges Wasser abdampft und das Produkt zu 3-mm-Strängen verformt. Diese Stränge werden bei 10°C getrocknet und bei 350°C calciniert. Katalysator D enthält 9,24 Gew.% B und 29,5 Gew.% P.

### Katalysator E

Katalysator E ist ein gefälltes Aluminiumphosphat, hergestellt durch Fällung aus Al(NO₃)₃ und H₃PO₄-Lösung mit NH₃ bei pH 6 bis 7. Nach Abfiltrieren wird der Niederschlag bei 110°C getrocknet und bei 500°C calciniert. Katalysator C enthält 22,7 Gew.% Al und 25,3 Gew.% P.

### Katalysator F

Katalysator F besteht aus pyrogenem TiO₂, das unter Zugabe eines Verformungshilfsmittels zu 2-mm-Strängen verformt, bei 110°C getrocknet und bei 500°C 16 h calciniert wird. Die Strangpreßlinge werden mit 15 %iger HCl (Massenverhältnis = 1 : 10) 1 h bei 80°C behandelt, danach chloridfrei gewaschen, bei 110°C getrocknet und 1 h bei 600°C calciniert.

### Katalysator G

Katalysator G besteht aus Katalysator B, der mit verdünnter Phosphorsäure getränkt, bei 130°C getrocknet und bei 540°C 2 h calciniert wird. Der P-Gehalt beträgt 4,73 Gew.%.

### Katalysator H

Katalysator H besteht aus handelsüblichem Nioboxydhydrat (61,2 Gew.% Nb; 9,5 Gew.% H₂O), das mit Verformungshilfsmittel zu 2-mm-Strängen verstrangt, bei 110°C getrocknet und bei 500°C 16 h calciniert wird.

### Katalysator I

Katalysator I besteht aus Katalysator B, der mit verdünnter NaH₂PO₄-Lösung getränkt, bei 110°C getrocknet und bei 500°C 14 h calciniert wird. Er enthält 6,2 Gew.% Na und 7,7 Gew.% P.

### Beispiele 1 bis 3

Ein Gemisch aus 2-Methylimidazol, Essigsäure und Essigsäureanhydrid (Molverhältnis = 1 : 4 : 1) wird verdampft und mit einem Stickstoffstrom von 6 l/h bei 450°C und einer Belastung von 4 h⁻¹ über den Katalysator geleitet.

Das Reaktionsprodukt wird in einer Glasapparatur kondensiert und gaschromatographisch analysiert.

**Tabelle 1**

| Acetylierung von 2-Methylimidazol | | | | | | | |
|---|---|---|---|---|---|---|---|
| Beispiel | Katalysator | Laufzeit [h] | Temp. [°C] | WHSV [h⁻¹] | GC-Analyse des Austrags*) | | |
| | | | | | 2-Methylimidazol [%] | N-Acetyl-2-methylimidazol [%] | 4-Acetyl-2-methylimidazol [%] |
| 1 | I | 6 | 450 | 4 | 19,5 | 0000 | 14,1 |
| 2 | C | 6 | 450 | 4 | 16,9 | 8,4 | 15,5 |
| 3 | D | 6 | 450 | 4 | 14,3 | 8,4 | 19,3 |

| | | | | | | | |
|---|---|---|---|---|---|---|---|
| *) gesammelter Austrag über 2 h nach 6 h Laufzeit. | | | | | | | |

### Beispiele 4 bis 10

Ein Gemiscch aus Imidazol, Essigsäure und Essigsäureanhydrid (Molverhältnis = 1 : 4 : 1) wird verdampft und mit einem Stickstoffstrom von 6 l/h bei 450°C und einer Belastung von 3 h⁻¹ über den Katalysator geleitet.

Das Reaktionsprodukt wird in einer Glasapparatur kondensiert und gaschromatographisch analysiert.

**Tabelle 2**

| Acetylierung von Imidazol | | | | | | | |
|---|---|---|---|---|---|---|---|
| Beispiel | Katalysator | Laufzeit [h] | Temp. [°C] | WHSV [h⁻¹] | GC-Analyse des Austrags*) | | |
| | | | | | Imidazol [%] | N-Acetylimidazol [%] | c-Acetylimidaol [%] |
| 4 | B | 6 | 450 | 3 | 3,6 | 31,7 | 1,3 |
| 5 | E | 6 | 450 | 3 | 15,8 | 12,8 | 3,1 |
| 6 | C | 6 | 450 | 3 | 22,4 | 6,2 | 2,0 |
| 7 | F | 6 | 450 | 3 | 25,7 | 0,2 | 1,4 |
| 8 | A | 6 | 450 | 3 | 19,0 | 3,8 | 3,0 |
| 9 | G | 6 | 450 | 2 | 6,2 | 15,0 | 2,0 |
| 10 | H | 6 | 450 | 3 | 12,2 | 9,7 | 1,2 |

| | | | | | | | |
|---|---|---|---|---|---|---|---|
| *) Mittelwerte über 6 h Laufzeit | | | | | | | |

### Beispiel 11

Die Reaktion wird in einem 1-Liter-Wirbelbettreaktor in derGasphase durchgeführt. Die Temperatur beträgt 400°C. Zugegeben werden 60 g/h eines Gemisches aus 2-Methylimidazol und Acetanhydrid (1 : 1,3 molar), als Wirbelgas werden 150 l/h Stickstoff zugeleitet. Der Reaktor ist mit 300 ml Katalysator in wirbelfähiger Form gefüllt.

Als Katalysator wird handelsübliches SiO₂ in wirbelfähiger Form verwendet.

Die Analyse des Reaktoraustrages ergibt einen Umsatz bezogen auf 2-Methylimidazol von 87% mit einer Selektivität von 75 % zu 4-Acetyl-2-methylimidazol.

## Patentansprüche

1. Verfahren zur Herstellung von acylierten Imidazolen der Formel I 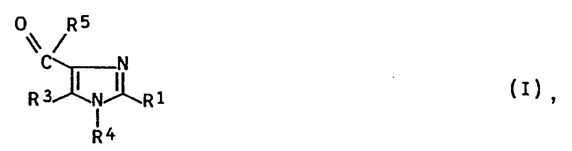 in der R¹ und R³ = H, Alkyl mit 1 bis 12 Kohlenstoffatomen, Alkenyl mit bis zu 12 Kohlenstoffatomen, Aryl- oder Aralkyl- oder Alkylarylreste,
R⁴ = H-, Alkyl mit 1 bis 12 Kohlenstoffatomen, Alkenyl mit bis zu 12 Kohlenstoffatomen, Aryl-, Aralkyl- oder Alkylarylreste, sowie R⁵ = Alkyl mit 1 bis 12 Kohlenstoffatomen, Alkenyl mit bis zu 12 Kohlenstoffatomen, Aryl-, Aralkyl-, Alkylaryl- oder _Carboxyreste bedeuten, dadurch gekennzeichnet, daß man Imidazole der Formel II 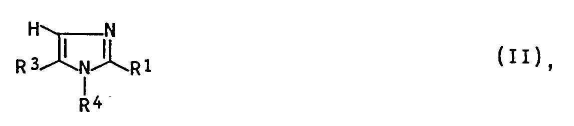 in der R¹ und R³ = Alkyl mit 1 bis 12 Kohlenstoffatomen, Alkenyl mit bis zu 12 Kohlenstoffatomen, Aryl=, Aralkyl- oder Alkylarylreste und R⁴ = H, Alkyl mit 1 bis 12 Kohlenstoffatomen, Alkenyl mit bis zu 12 Kohlenstoffatomen, Aryl-, Aralkyl- oder Alkylarylreste bedeuten, mit gängigen Acylierungsmitteln der Formel (III) 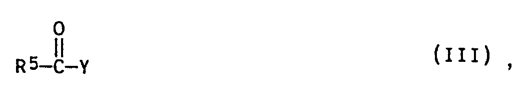 in der R⁵ die obengenannte Bedeutung hat und Y = Halogenid, Alkoxy-, Acyloxy- oder Hydroxyreste bedeutet, in Gegenwart von sauren Metalloxiden der zweiten bis vierten Haupt- und dritten bis sechsten Nebengruppe oder deren Gemische, die gegebenenfalls durch Phosphor- oder Borsäure getränkt sind, ohne Zeolithstruktur und/oder Aluminiumphosphate, Siliciumaluminiumphosphate, Siliciumeisenaluminiumphosphate, Cerphosphate, Zirkonphosphate, Borphosphate, Eisenphosphate, Strontiumphosphate oder deren Gemische in der Gasphase umsetzt.

2. Verfahren nach Anspruch 1, dadurch gekennzeichnet, daß man als Katalysatoren der Metalle B, Al, Si, Ga, Mg, Ca, Ce, La, Fe und Zr verwendet.

3. Verfahren nach Anspruch 1 oder 2, dadurch gekennzeichnet, daß man Katalysatoren verwendet, die mit phosphorhaltigen Substanzen behandelt werden.

4. Verfahren nach Anspruch 1 oder 2, dadurch gekennzeichnet, daß man Katalysatoren verwendet, die mit Mineralsäuren behandelt wurden.

## Claims

1. A process for preparing acylated imidazoles of the formula I 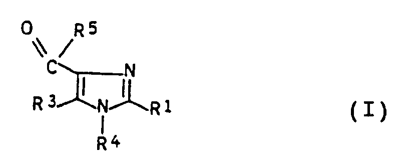 where R¹ and R³ are each H, alkyl of from 1 to 12 carbon atoms, alkenyl of up to 12 carbon atoms, aryl, aralkyl or alkylaryl, R⁴ is H, alkyl of from 1 to 12 carbon atoms, alkenyl of up to 12 carbon atoms, aryl, aralkyl or alkylaryl, and R⁵ is alkyl of from 1 to 12 carbon atoms, alkenyl of up to 12 carbon atoms, aryl, aralkyl, alkylaryl or carboxyl, which comprises reacting an imidazole of the formula II 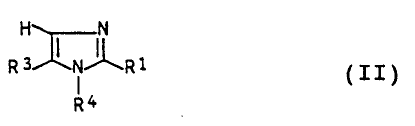 where R¹ and R³ are each alkyl of from 1 to 12 carbon atoms, alkenyl of up to 12 carbon atoms, aryl, aralkyl or alkylaryl, and R⁴ is H, alkyl of from 1 to 12 carbon atoms, alkenyl of up to 12 carbon atoms, aryl, aralkyl or alkylaryl, with a widely used acylating agent of the formula (III) 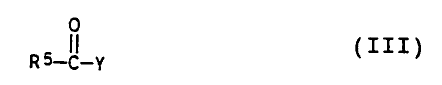 where R⁵ is as defined above and Y is halide, alkoxy, acyloxy or hydroxyl, in the gas phase in the presence of acidic metal oxides of the second to fourth main group or the third to sixth subgroup or mixtures thereof, which acidic metal oxides have no zeolite structure and may have been impregnated with phosphoric or boric acid, and/or aluminum phosphates, silicon aluminum phosphates, silicon iron aluminum phosphates, cerium phosphates, zirconium phosphates, boron phosphates, iron phosphates, strontium phosphates or mixtures thereof.

2. A process as claimed in claim 1, wherein the catalyst used is one of the metals B, Al, Si, Ga, Mg, Ca, Ce, La, Fe and Zr.

3. A process as claimed in claim 1 or 2, wherein the catalyst used has been treated with a phosphorus-containing substance.

4. A process as claimed in claim 1 or 2, wherein the catalyst used has been treated with a mineral acid.

## Revendications

1. Procédé de fabrication d'imidazoles acylés de formule (I) 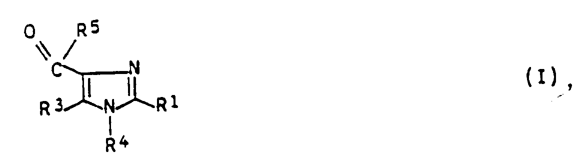 dans laquelle R¹ et R³ = H, alkyle à 1 à 12 atomes de carbone, alcényle comportant jusqu'à 12 atomes de carbone, restes aryle ou aralkyle ou alkylaryle,
R⁴ = H, alkyle à 1 a 12 atomes de carbone, alcényle comportant jusqu'à 12 atomes de carbone, restes aryle, aralkyle ou alkylaryle, et R⁵ = alkyle à 1 à 12 atomes de carbone, alcényle comportant jusqu'à 12 atomes de carbone, restes aryle, aralkyle, alkylaryle ou carboxyle, caractérisé en ce que l'on fait réagir en phase gazeuse un imidazole de formule (II) 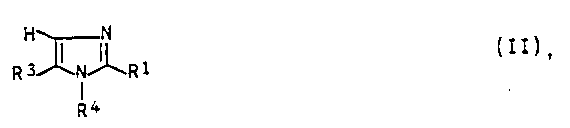 dans laquelle R¹ et R³ = alkyle à 1 à 12 atomes de carbone, alcényle comportant jusqu'à 12 atomes de carbone, restes aryle, aralkyle ou alkylaryle et R⁴ = H, alkyle à 1 à 12 atomes de carbone, alcényle comportant jusqu'à 12 atomes de carbone, restes aryle, aralkyle ou alkylaryle, avec des agents d'acylation habituels de formule (III) 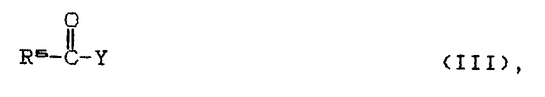 dans laquelle R⁵ a la signification indiquée ci-dessus et Y = un reste halogénure, alcoxy, acyloxy ou hydroxy, en présence d'oxydes' métalliques acides d'éléments des deuxième à quatrième groupes principaux et des troisième à sixièmesous-groupes, ou de leurs mélanges, qui sont le cas échéant imprégnés d'acide phosphorique ou borique, en l'absence d'une structure zéolitique et/ou de phosphate d'aluminium, de phosphate de silicium-aluminium, de phosphate de silicium-fer-aluminium, de phosphate de cérium, de phosphate de zirconium, de phosphate de bore, de phosphate de fer, de phosphate de strontium ou de leurs mélanges.

2. Procédé selon la revendication 1, caractérisé en ce que l'on utilise des catalyseurs des métaux B, Al, Si, Ga, Mg, Ca, Ce, La, Fe et Zr.

3. Procédé selon la revendication 1 ou 2, caractérisé en ce que l'on utilise des catalyseurs qui sont traités par des substances contenant du phosphore.

4. Procédé selon la revendication 1 ou 2, caractérisé en ce que l'on utilise des catalyseurs qui sont traités par des acides minéraux.
